Europäisches Patentamt

European Patent Office

Office européen des brevets

(11) Publication number: **0 134 605**
**B1**

(12) **EUROPEAN PATENT SPECIFICATION**

(45) Date of publication of patent specification: **15.03.89**

(51) Int. Cl.⁴: **G 01 N 33/53**

(21) Application number: **84201067.0**

(22) Date of filing: **22.12.81**

(60) Publication number of the earlier application in accordance with Art. 76 EPC: **0 067 182**

(54) Device for detecting antigens and antibodies.

(30) Priority: **22.12.80 AU 7043/80**

(43) Date of publication of application:
**20.03.85 Bulletin 85/12**

(45) Publication of the grant of the patent:
**15.03.89 Bulletin 89/11**

(84) Designated Contracting States:
**AT BE CH DE FR GB LI LU NL SE**

(56) References cited:
**DE-A-1 598 224**
**FR-A-2 417 094**
**GB-A-2 015 158**

(73) Proprietor: **COMMONWEALTH SERUM LABORATORIES COMMISSION**
**45 Poplar Road**
**Parkville, Vic. 3052 (AU)**

(72) Inventor: **Chandler, Howard Milne**
**Menzies Road**
**Kangaroo Ground Victoria 3097 (AU)**
Inventor: **Healey, Kevin**
**44 Hilbert Road**
**Niddrie Victoria 3042 (AU)**
Inventor: **Hurrell, John Gordon Rowan**
**32 Dublin Avenue**
**Strathmore Victoria 3041 (AU)**

(74) Representative: **Bizley, Richard Edward et al**
**BOULT, WADE & TENNANT 27 Furnival Street**
**London EC4A 1PQ (GB)**

Courier Press, Leamington Spa, England.

## Description

This invention relates to improvements in apparatus for performing enzyme-linked immunosorbent assays for the detection and quantitative determination of antigens and antibodies. In particular, the present invention relates to improvements in apparatus which enable such assays to be performed by following a simple procedure which does not require the use of elaborate laboratory equipment or trained laboratory personnel, and hence may be performed under relatively adverse conditions, for example in the field.

In prior Australian Patent Specification AU—B—540523, there are described improved methods and apparatus for the performance of enzyme-linked immunosorbent assays (ELISA), which are particularly described with reference to their use in the detection of anti-tetanus antibodies in sheep and the detection and identification of snake venoms as examples of such assays.

The prior specification describes apparatus for detecting or determining the presence of antigenic or haptenic substances or antibodies in a sample which comprises a plurality of containers, one of said containers being provided with a cap which has antibodies or antigenic or haptenic substances attached to an internal surface thereof, said cap being adapted to be transferred from one to another of said containers so the internal surface thereof is brought into contact with solutions contained in said plurality of containers in a predetermined sequence. In one embodiment the "internal surface" of the cap includes those surfaces of the cap themselves which, when the cap is applied to a container, are exposed to the contents of the container, as well as the surfaces of projecting portions provided on the cap to extend within the body of the container when the cap is applied thereto, and surfaces which are physically attached to or retained within a cap which are themselves exposed to the contents of the container when the cap is applied thereto. In an alternative embodiment, the "internal surface" of the cap to which the antibodies or antigenic or haptenic substances are attached comprises the internal surface of a tube attached to the cap, for example the tube portion of a "dropper-type" cap having a rubber teat or the like to enable the fluid to be drawn up into the tube and expelled therefrom.

One of the main limitations of the apparatus disclosed in the prior specification lies in the fact that only a single antibody or antigenic or haptenic substance can be attached to the internal surface of the cap or of the tube attached to the cap. This is a relatively serious limitation when the apparatus is to be used in a screening-type test. For example, in the detection and identification of snake venoms in Australia, it is usually necessary to be able to detect at least two and in most cases five primary snake venoms to enable selection of the appropriate antivenom. This means that when using the apparatus disclosed in the prior specification, at least two and often five separate tests must be carried out in order to identify the unknown venom in the clinical sample, together with control tests where appropriate. The necessity to carry out a number of separate tests is, of course, particularly disadvantageous from the point of view of carrying out the necessary tests in the field, and it is an object of the present invention to provide a further improvement in this apparatus whereby the requirement for performing a plurality of separate tests may be avoided.

GB—A—2015158 discloses a device which enables a simultaneous determination of several substances to be performed from a single sample. However GB—A—2015158 does not disclose the use of capillary tubes and further it requires that the tubes be mutually detachably connected.

According to the present invention there is provided a device for use in detecting or determining the presence of antigenic or haptenic substances or antibodies in a sample, which device comprises a plurality of capillary elements, each of said capillary elements having antibodies or antigenic or haptenic substances attached to an internal surface thereof, and means for causing fluids to pass simultaneously or sequentially through said plurality of capillary elements.

The invention further includes a test kit for use in detecting or determining the presence of antigenic or haptenic substances or antibodies in a sample by the enzyme-linked immunosorbent assay technique, which comprises:

(i) a plurality of capillary elements, each of said capillary elements having antibodies or antigenic or haptenic substances attached to an internal surface thereof, and means for causing fluids to pass simultaneously or sequentially through said plurality of capillary elements;

(ii) antibody-enzyme or antigen-enzyme conjugate; and

(iii) an enzyme substrate/indicator system.

In use of the device of the invention, fluids including unknown samples and test reagents may be drawn into the device and hence into contact with the internal surface of each of the capillary elements, and subsequently expelled therefrom. By way of example, such means may comprise a rubber teat or a syringe device.

The device of the present invention is particularly intended for use with test apparatus of the type generally disclosed in the prior Australian Specifications referred to above. Thus, the device of the present invention may be provided in combination with a plurality of containers whereby an assay may be performed by bringing the internal surfaces of the capillary elements of the device into contact with solutions contained in said plurality of containers in a predetermined sequence. Alternatively, the various test solutions and reagents may be provided in the wells of a reagent tray.

In general, the capillary elements comprising the device of the present invention may be made of any suitable material such as glass, polyvinyl chloride, polystyrene or other suitable plastics materials, however it is important that the elements be transparent so that reactions taking place within each element may be observed externally. The antibodies or antigenic or haptenic substances may be attached to the internal surface of the capillary elements by known techniques, for example, in the case of glass capillary elements, by adsorption or covalent bonding, and in the case of elements of plastics materials, by adsorption or covalent bonding. By way of example, the capillary elements may be 1.5 cm to 2.0 cm in length, and have an internal diameter of about 1 mm, and an external diameter of about 2 mm. Such elements have a capacity of around 5—20 µl.

In one embodiment of the device of this invention, the plurality of capillary elements are connected in series by tubular connecting elements. In this embodiment, fluids such as unknown samples and test reagents are drawn into and expelled from the elements in sequence. The tubular connecting elements inter-connecting the individual tubular elements may be of any suitable material, and by way of example, silicone rubber and polyvinyl chloride have been found to be suitable materials.

It will be appreciated that in general terms, the use of a device of the present invention enables a single sample to be "screened" against a number of known antibodies or antigenic or haptenic substances in a single test sequence. In such a "screening" assay, each capillary element will be provided with a different known antibody or antigenic or haptenic substance, and a "positive control" capillary element may also be included in the device. In order to test the unknown sample against each of the antibodies or antigenic or haptenic substances attached to the internal surfaces of the capillary elements, it is only necessary to perform a single test sequence by drawing the various test fluids in sequence into the device of the present invention so that each fluid passes into all the capillary elements before it is expelled from the device. One particular example of the use of the device of the present invention in this manner in the performance of an ELISA assay for the detection of snake venoms will be descriebd in detail hereinafter.

Alternatively, the device of this invention may be used in a semi-quantitative type of "screening" test in which a single unknown sample is tested alongside one or more known positive controls against a single antibody or antigenic or haptenic substance in a single test sequence. The known positive control or controls may have known levels of the material which is to be detected in the sample, and once again the single test sequence is performed by drawing the various test fluids in sequence into the device and hence into the capillary elements before being expelled therefrom. The use of this device in this manner in the performance of digoxin and tetanus screening tests is described below as further examples of the use of this device.

The device of the invention may be used to carry out an improved method of detecting or determining the presence of antibodies or of antigenic or haptenic substances in a sample which is claimed in our copending application EP—A—0067182, from which the present application is divided.

Our copending application claims a method of detecting or determining the presence of antibodies or an antigenic or haptenic substance in a sample by the enzyme-linked immunosorbent assay technique in which the binding of an antibody-enzyme or antigen-enzyme conjugate to a solid phase is used to indicate the presence or absence of antibodies or antigenic or haptenic substance in said sample, characterised in that the enzyme in said conjugate is urease, the solid phase is contacted with urea as the enzyme substrate, and the presence of ammonia is detected or determined using di-bromo-o-cresol-sulfonphthalein (also known as Bromcresol Purple) to indicate the presence of antibodies or antigenic or haptenic substances in the sample.

Our copending application EP—A—0067182 also claims a test kit for the detection or determination of the presence of antibodies or an antigenic or haptenic substance in a sample by the enzyme-linked immunosorbant assay technique in which the binding of an antibody-enzyme or antigen-enzyme conjugate to a solid phase is used to indicate the presence or absence of antibodies or antigenic or haptenic substances in said sample, said kit comprising an antibody-enzyme or antigen-enzyme conjugate, the enzyme in said conjugate being urease, and a substrate/indicator system comprising urea as the enzyme substrate and di-bromo-o-cresolsulfon-phthalein as indicator.

The present invention is illustrated, by way of example, with reference to the accompanying drawings, in which:

Figure 2 is a part-sectional view through a first embodiment of a device in accordance with the invention; and

Figure 3 is a part-sectional view of a second embodiment of a device in accordance with the invention.

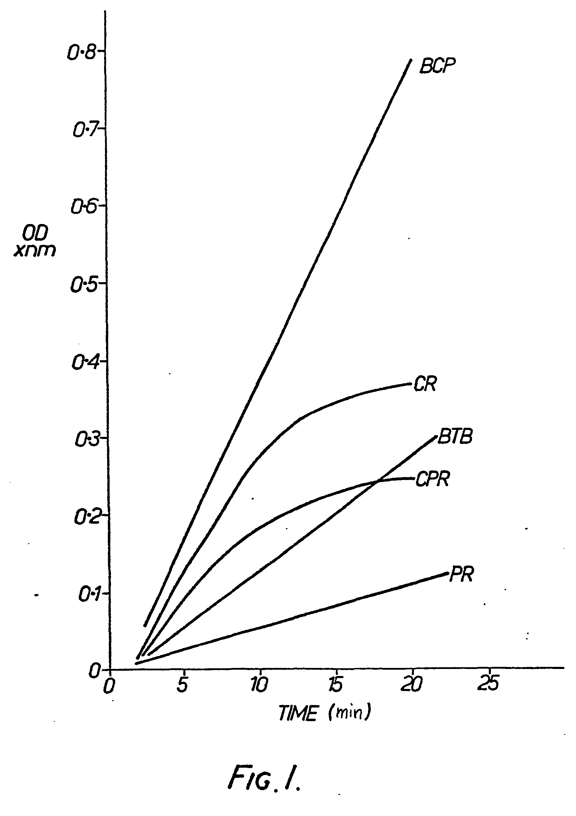

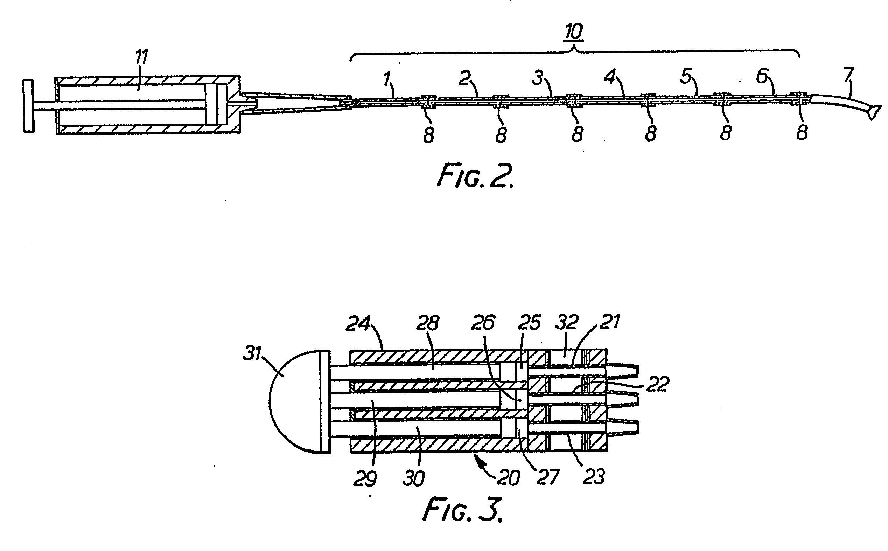

The device depicted in Figure 2 is designed particularly for use as a snake venom detection apparatus, and will be described hereinafter with particular reference to its use in this manner. The device comprises a glass tube assembly 10, and a syringe 11 which is arranged to draw fluids into and expel fluids from the glass tube assembly 10. Assembly 10 comprises six tubular elements 1, 2, 3, 4, 5, 6, which are joined in series by pieces of silicone rubber tubing 8. Each of the tubes 1 to 5 has a different antivenom adhering to its internal surface as follows:—

Tube 1 Tiger Snake
Tube 2 Brown Snake
Tube 3 King Brown Snake
Tube 4 Death Adder
Tube 5 Taipan

In addition, tube 6 is provided as a control positive tube. The free end of tube 6 is sealed by means of a sealed plastic tube 7 which, in contrast to the transparent tubes 1 to 5, may be coloured, or opaque. The device illustrated in Figure 2 of the drawings is used in the detection and identification of snake venoms in combination with a number of solution-containing bottles as follows:—

Bottle 1 Contains washing buffer

Bottle 2 Contains antibody-enzyme conjugate, i.e. antivenom coupled to urease, in buffered saline

Bottle 3 Contains washing liquid (unbuffered)

Bottle 4 Contains enzyme substrate and indicator, i.e. urea solution, Bromcresol Purple and EDTA (to complex any metal ions which may poison the urease).

The following procedure is followed in performing the test:

(1) Check the joints between tubes of the device are secure. Immediately before use cut sealed tip from plastic tube and expel liquid from tubes.

(2) Draw clinical sample until tube assembly filled.

(3) Allow to rest at room temperature for approximately 10 minutes.

(4) Expel contents (waste). Draw in wash *(Bottle 1)* until syringe half full (approx.). Expel (waste). Repeat 3 times.

(5) Draw air, followed by conjugate *(Bottle 2)*, until all tubes filled.

(6) Allow to rest at room temperature for approximately 10 minutes.

(7) Expel contents (waste). Draw in wash *(Bottle 3)* until synringe half full (approx.). Expel (waste). Repeat 3 times.

(8) Draw air, followed by substrate *(Bottle 4)*, until all tubes filled.

(9) Observe carefully against a white background for 20 minutes from step 8. All snake venoms cross-react immunologically therefore note the *first* tube to follow the colour change sequence of yellow-green-grey-blue-purple shown by the positive control tube 6.

It will be noted that in the test described above, the indicator used to detect the production of ammonia in the urease/urea system is Bromcresol Purple. As is described in our copending application, this indicator has been found to be of particular benefit in this system and it is found that the detection limit of the test as performed above is approximately 10 to 20 nanograms/ml of sample used in step 2. Furthermore, greater sensitivity may be achieved by repeating the test using longer incubations for steps 3 and 6 (for example of the order of 20 to 30 minutes).

The device described in detail above is not restricted to use in the detection of snake venoms, and it has application in the detection of tetanus antibodies as described in the prior specifications referred to above, as well as in other screening tests utilising the ELISA technique.

The device depicted in Figure 3 illustrates an alternative embodiment of the device of the invention for use, for example, in a digoxin or tetanus screening test, and will be described hereinafter with particular reference to its use in this manner.

The device 20 comprises three glass tubular or capillary elements 21, 22 and 23 mounted in a common body or support 24. Each of these elements has a haptenic substance adhering to its internal surface as described below. Support 24 is also provided with three bores 25, 26 and 27 which communicate with elements 21, 22 and 23, respectively. Plungers 28, 29 and 30 are located within bores 25, 26 and 27, respectively, and are adapted to draw fluids through the respective capillary elements and into the bores, and to express these fluids out through the respective elements on movement of the plungers within the bores. Plungers 28, 29 and 30 are interconnected by a single handle piece 31 so that fluids are simultaneously drawn into and expressed from each of the capillary elements 21, 22 and 23. A white background area 32 is provided behind a portion of elements 21, 22 and 23 to assist in visual comparison of colour development in these elements.

The device 20 may be used in a digoxin screening test based on the inhibition of urease-anti-digoxin antibody conjugate binding to digoxin immobilised on the inner surface of the glass capillary elements caused by free digoxin present in the unknown serum. This test may be designed to detect digoxin in the serum of patients at levels below 1.0 nanograms/ml, between 1.0 and 3.0 nanograms/ml, and above 3.0 nanomgrams/ml. The accepted therapeutic range for digoxin is between 1.0 and 3.0 nanograms/ml, serum levels below this range may be ineffective, serum levels above this range may be toxic. This simple screening test enables the physician to estimate patient compliance with prescribed medication and, in cases of suspected toxicity, to rapidly confirm the diagnosis.

Due to the haptenic nature of digoxin, this test works on the inhibition of colour development which is in contrast to the snake venom detection test described above. The unknown sample of patient's serum and urease-anti-digoxin conjugate are preincubated together in a test tube or well of a reagent tray. Simultaneously known positive serum samples (1 nanogram/ml and 3 nanogram/ml free digoxin) are pre-incubated with conjugate in adjacent tubes or wells. After a predetermined incubation period at room temperature, the mixtures are simultaneously drawn into respective capillary elements 21, 22 and 23, each of which has digoxin conjugated to human serum albumin covalently attached to the inner surface thereof. After a further incubation period, the mixtures are expressed out of the elements 21, 22 and 23, the elements are washed and the urea indicator previously described simultaneously drawn into the elements. The amount of inhibition of colour development in the case of the unknown sample is then compared to that seen in the cases of the sera containing known amounts of free digoxin to provide a semi-quantitative assay of the unknown sample.

An alternative use of the device 20 is in a tetanus screening assay in which a sample of the blood or serum of a patient is screened against reference serums or blood to ascertain the level of tetanus antibodies in the sample. The assay is based on detection of tetanus antibodies binding to purified tetanus antigen immobilised on the inner surfaces of the glass capillary elements, the procedure comprising incubating the sample serum or blood and high (for example at least 1.28 International Units/ml) and low (for example no greater than 0.01 International Units/ml) reference sera or bloods within individual elements 21, 22 and 23 of the device 20 for a period of about 10 minutes, and after appropriate washing introducing anti-human Ab(IgG)—urease conjugate and incubating for a further period of about 10 minutes. After further washing the substrate/indicator system comprising urea and Bromcresol Purple as previously described is introduced and the colour development in the sample test compared with that in the high and low reference tests to indicate the tetanus antibody titre in the sample.

By using the assay outlined above, the sample can be classified into one of three categories:

(a) A titre equal to or greater than the high reference titre

—indicates patient highly immune and probably does not require booster vaccination (which would possibly involve risk of adverse reaction).

(b) A titre between the high and low reference titres

—indicates patient immune but a booster vaccination probably warranted.

(c) A titre equal to or less than the low reference titre

—indicates patient non-immune and in cases of injury administration of tetanus immune globulin and vaccine probably warranted.

In each of the examples set out above, the urease conjugate is prepared by standard procedures, either by the single step glutaraldehyde method (Aurameas, S., Ternynck, T. and Guesdon, J. L., "Coupling of enzymes to antibodies and antigens", 1978 Scand, J. Immunol, 8 (Suppl. 7), pp. 7—23) or by the M.B.S. method (Monji, N., Malkus, H. and Castro, A., "Maleimide derivative of hapten for coupling to enzyme: A new method in enzyme immunoassay" Biochem. Biophys. Res. Comm., 85, 671 (1978)). The conjugate reagent is made up in buffered saline (pH 7.2) as described in detail above, and includes ovalbumin in an amount of from 0.25% to 1% by weight to minimise non-specific binding of the conjugate.

The substrate solution comprises a dilute urea solution, for example containing 1 mg/ml of urea in glass distilled water, to which is added EDTA to complex heavy metal ions which may poison the urease.

## Claims

1. A device for use in detecting or determining the presence of antigenic or haptenic substances or antibodies in a sample, which device comprises a plurality of capillary elements, each of said capillary elements having antibodies or antigenic or haptenic substances attached to an internal surface thereof, and means for causing fluids to pass simultaneously or sequentially through said plurality of capillary elements.

2. A device according to claim 1, characterised in that said capillary elements are connected in series by tubular connecting elements, and said series of elements is connected to a single means for causing fluids to pass therethrough.

3. A device according to claim 2, characterised in that said single means for causing fluids to pass through said series of capillary elements comprises a syringe device having a bore in fluid connection with an end one of said series of capillary elements, and a plunger movable within said bore.

4. A device according to claim 1, characterised in that said capillary elements are mounted side-by-side in a support and in fluid connection to individual bores within said support, and said means for causing fluids to pass through said capillary elements comprises means for simultaneously drawing fluids into and expressing said fluids out of the bores through the respective capillary elements.

5. A device according to claim 4, characterised in that individual plungers are mounted within said bores for movement relative thereto to draw fluids into and express fluids out of said bores, and said individual plungers are interconnected for simultaneous movement relative to the respective bores.

6. A device according to any one of claims 1 to 5, characterised in that said capillary elements are constructed of glass or plastics materials, and said antibodies or haptenic or antigenic substances are attached to the internal surfaces thereof by adsorption or covalent bonding.

7. A test kit for use in detecting or determining the presence of antigenic or haptenic substances or antibodies in a sample by the enzyme-linked immunosorbent assay technique, which comprises:

(i) a plurality of capillary elements, each of said capillary elements having antibodies or antigenic or haptenic substances attached to an internal surface thereof, and means for causing fluids to pass simultaneously or sequentially through said plurality of capillary elements;

(ii) antibody-enzyme or antigen-enzyme conjugate; and

(iii) an enzyme substrate/indicator system.

8. A test kit according to claim 7, wherein the capillary elements and the means for causing fluids to pass through the elements are as characterised in any one of claims 2 to 6.

9. A test kit according to claim 7 or claim 8, wherein the enzyme in the conjugate is urease and the indicator is di-bromo-o-cresolsulfonphthalein.

**Patentansprüche**

1. Vorrichtung zur Verwendung bei der Feststellung oder Bestimmung des Vorhandenseins von antigenen oder haptenen Substanzen oder Antikörpern in einer Probe, wobei die Vorrichtung eine Anzahl von Kapillarelementen, die jeweils an einer inneren Oberfläche angebrachte Antikörper oder antigene oder haptene Substanzen aufweisen, und eine Einrichtung enthält, die veranlaßt, daß gleichzeitig oder aufeinanderfolgend Fluide durch die Anzahl von Kapillarelementen strömen.

2. Vorrichtung nach Anspruch 1, dadurch gekennzeichnet, daß die Kapillarelemente in einer Reihe durch rohrförmige Verbindungselemente verbunden sind, und daß die Reihe von Elementen mit einer einzigen Einrichtung verbunden ist, die veranlaßt, daß Fluide dadurch strömen.

3. Vorrichtung nach Anspruch 2, dadurch gekennzeichnet, daß die einzige Einrichtung, die veranlaßt, daß durch die Reihe von Kapillarelementen Fluide strömen, einen Spritzenkörper mit einer Bohrung, die in Fluidverbindung mit einem Ende der Reihe von Kapillarelementen steht, und einen in der Bohrung beweglichen Kolben aufweist.

4. Vorrichtung nach Anspruch 1, dadurch gekennzeichnet, daß die Kapillarelemente Seite an Seite in einer Halterung angeordnet sind und in Fluidkontakt mit einzenen Bohrungen in der Halterung stehen, und daß die Einrichtung, die veranlaßt, daß Fluide durch die Kapillarelemente strömen, eine Einrichtung zum gleichzeitigen Einziehen von Fluiden in und Hinausdrücken der Fluide aus den Bohrungen durch die jeweiligen Kapillarelemente aufweist.

5. Vorrichtung nach Anspruch 4, dadurch gekennzeichnet, daß einzelne Kolben in den Bohrungen für eine Relativbewegung dazu angeordnet sind, um Fluide in die Bohrungen einzuziehen und Fluide aus den Bohrungen hinauszudrücken, und daß die einzelnen Kolben für eine gleichzeitige Bewegung relativ zu den jeweiligen Bohrungen miteinander verbunden sind.

6. Vorrichtung nach einem der Ansprüche 1 bis 5, dadurch gekennzeichnet, daß die Kapillarelemente aus Glas oder Kunststoffmaterial ausgebildet sind, und daß die Antikörper oder haptenen oder antigenen Substanzen durch Adsorption oder eine kovalente Bindung an den inneren Oberflächen davon angebracht sind.

7. Testausrüstung zur Verwendung bei der Feststellung oder Bestimmung des Vorhandenseins von antigenen oder haptenen Substanzen oder Antikörpern in einer Probe mittels der Enzym-verbundenen Immunsorbens-Untersuchungstechnik, die enthält:

(i) eine Anzahl von Kapillarelementen, wobei jedes Kapillarelement an einer inneren Oberfläche angebrachte Antikörper oder antigene oder haptene Substanzen aufweist, und eine Einrichtung, die veranlaßt, daß Fluide gleichzeitig oder aufeinanderfolgend durch die Anzahl von Kapillarelementen strömen;

(ii) ein Antikörper-Enzym- oder Antigen-Enzym-Konjugat; und

(iii) ein Enzym-Substrat/Indikator-System.

8. Testausrüstung nach Anspruch 7, wobei die Kapillarelemente und die Einrichtung, die veranlaßt, daß Fluide durch die Elemente strömen, wie in einem der Ansprüche 2 bis 6 gekennzeichnet sind.

9. Testausrüstung nach Anspruch 7 oder Anspruch 8, wobei das Enzym im Konjugat Urease ist und der Indikator Dibromokresolsulfonphthalein.

**Revendications**

1. Dispositif de détection ou de détermination de la présence de substances antigéniques ou hapténiques, ou d'anticorps dans un échantillon, ce dispositif comprenant plusieurs éléments capillaires, des anticorps, ou des substances antigéniques ou hapténiques étant fixés sur une surface interne de chacun desdits éléments capillaires, et des moyens pour faire passer simultanément ou successivement des fluides à travers les différents éléments capillaires.

2. Dispositif selon la revendication 1, caractérisé en ce que lesdits éléments capillaires sont reliés en série par des raccords tubulaires, et en ce que ladite série d'éléments est reliée à un seul organe destiné à faire passer des fluides dans ceux-ci.

3. Dispositif selon la revendication 2, caractérisé en ce que ce seul organe destiné à faire passer des fluides à travers la série d'éléments capillaires, comprend une seringue comportant un alésage en communication de fluide avec une extrémité de ladite série d'éléments capillaires, et un piston déplaçable à l'intérieur dudit alésage.

4. Dispositif selon la revendication 1, caractérisé en ce que lesdits éléments capillaires sont disposés côte à côte sur un support en étant en communication de fluide avec des alésages individuels ménagés à l'intérieur du support, et en ce qu'il est prévu des moyens pour faire passer des fluides à travers lesdits éléments capillaires, comprenant des moyens pour introduire simultanément des fluides dans les alésages et les en expulser à travers les éléments capillaires respectifs.

5. Dispositif selon la revendication 4, caractérisé en ce que les pistons individuels sont montés déplaçables dans lesdits alésages afin d'aspirer des fluides dans lesdits alésages et de les en expulser et en ce que les pistons sont reliés pour permettre un déplacement relatif simultané dans les alésages respectifs.

6. Dispositif selon l'une quelconque des revendications 1 à 5, caractérisé en ce que lesdits éléments capillaires sont réalisés en verre ou en matière plastique, et en ce que les anticorps, ou les substances hapténiques ou antigéniques, sont fixés sur leurs surfaces internes par adsorption ou liaison covalente.

7. Nécessaire d'essai pour détecter ou déterminer la présence de substances antigéniques ou hepténiques, ou d'anticorps dans un échantillon selon la technique d'essai par immunosorbant lié à une enzyme, comprenant:

(i) plusieurs éléments capillaires, des anticorps, des substances antigéniques ou hapténiques étant fixés sur une surface interne de chacun de ces éléments capillaires, et des moyens pour faire passer simultanément ou successivement des fluides à travers les différents éléments capillaires;

(ii) un conjugué anticorps-enzyme ou antigène-enzyme; et

(iii) un système de substrat enzymatique et d'indicateur.

8. Nécessaire d'essai selon la revendication 7, dans lequel les éléments capillaires et les moyens destinés à faire passer des fluides à travers les éléments, sont tels que définis selon l'une quelconque des revendications 2 à 6.

9. Nécassaire d'essai selon la revendication 7 ou la revendication 8, dans lequel dans le conjugué, est l'uréase, et en ce que l'indicateur est la dibromo-o-crésolsulfonephtaléine.

FIG. 1.

FIG. 2.

FIG. 3.

EP 0 134 605 B1